# EUROPÄISCHE PATENTANMELDUNG

(11) **EP 4 588 501 A1**
(43) Veröffentlichungstag der Anmeldung: **23.07.2025**
(21) Anmeldenummer: 24153240.7
(22) Anmeldetag: 22.01.2024
(51) Int. Cl.: A61M 60/422, A61M 60/822, A61M 60/855, H02P 6/185, H02P 21/18

(54) **PUMPE MIT EINER STEUERVORRICHTUNG SOWIE VERFAHREN ZUR ERMITTLUNG DER POSITION EINES ROTORS**

(71) Anmelder: Berlin Heart GmbH, 12247 Berlin (DE)
(72) Erfinder: PETERS, Oliver, 12247 Berlin (DE); BYKOV, Valentin, 12247 Berlin (DE); JAHNE, Dr.-Ing. Helmut, 12247 Berlin (DE)
(74) Vertreter: Pfenning, Meinig & Partner mbB

(57) **Zusammenfassung**

Die Erfindung bezieht sich auf eine Pumpe, umfassend eine Pumpenkammer (2), in der ein in wenigstens einem Lager (14, 14', 15, 15') um eine Rotationsachse (3) drehbar gelagerter Rotor (1) mit wenigstens einem Rotormagnet (8, 9) und mit wenigstens einem Förderelement (1a, 1b) zur Förderung von einer Flüssigkeit, insbesondere Blut, angeordnet ist, einen Stator mit mehreren Antriebsspulen (5, 6) sowie eine Steuereinrichtung (7), wobei der Rotor ein wenigstens teilweise elektrisch leitendes Target (18) in Form eines Körpers aufweist, der von Mess-Wechselmagnetfeldern durchsetzbar ist, die durch eine Wechselmagnetfeldquelle erzeugt werden, dass die Pumpe eine oder mehrere Empfangsspulen aufweist und dass die Steuereinrichtung dazu eingerichtet ist, aus jeweiligen Wechselspannungen und Wechselströmen eine Position des Rotors (1) zu ermitteln. Weiter bezieht sich die Erfindung auch auf ein Verfahren zur Positionsbestimmung des Rotors.

## Beschreibung

Die Erfindung liegt auf dem Gebiet der Sensorik, insbesondere des Maschinenbaus, der Elektronik und der Medizintechnik. Bevorzugte Anwendungen sind Pumpen und Motoren.

Viele Pumpen weisen motorisch antreibbare Rotoren auf, die Förderelemente für die zu fördernde Flüssigkeiten aufweisen oder tragen. Derartige Rotoren sind oft von den zu fördernden Flüssigkeiten umgeben und kommen jedenfalls mit diesen in Berührung. Je nach der Pumpenlast wirken auf einen Pumpenrotor veränderliche Kräfte. Grundsätzlich ist es aus verschiedenen Gründen wünschenswert, eine Position eines Pumpenrotors möglichst zuverlässig und mit vertretbarem Aufwand zu ermitteln. Dies ist insbesondere bei pneumatisch, hydraulisch oder magnetisch gelagerten Rotoren von herausragendem Interesse.

Zu diesem Zweck werden oft Magnetfeldsensoren, beispielsweise Hall- Sensoren eingesetzt, die aufgrund magnetischer Wechselwirkung ermöglichen, eine Position eines mit dem Sensor wechselwirkenden Bauteils relativ zu dem Sensor zu ermitteln. Auch die Verwendung von Wirbelstromsensoren zur Positionsmessung ist grundsätzlich bekannt.

Beispielsweise ist aus der europäischen Patentschrift EP2507613 B1 eine Blutpumpe mit einem Rotor bekannt, bei der mittels mehrerer Wirbelstromsensoren eine Position des Rotors in der Axialrichtung, um die der Rotor im Betrieb rotiert, bestimmt wird. Nachteilig sind hierbei insbesondere der zusätzliche Bauraum und die zusätzlichen Anschlussleitungen der Sensorspulen.

Aus dem Dokument US2022/0409878 A1 sind bereits verschiedene Maßnahmen und Methoden zur Positionsmessung eines Rotors in einer Blutpumpe bekannt.

Vor dem Hintergrund des Standes der Technik liegt der vorliegenden Erfindung die Aufgabe zugrunde, eine Pumpe mit einem Rotor zu schaffen, bei der sich die Position des Rotors möglichst zuverlässig mit geringem Aufwand ermitteln lässt.

Die Aufgabe wird durch die Erfindung mit den Merkmalen der unabhängigen Patentansprüche gelöst. Die abhängigen Patentansprüche stellen mögliche Implementierungen vor.

Die Erfindung bezieht sich unter anderem auf eine Pumpe, insbesondere Blutpumpe, umfassend
eine Pumpenkammer, in der ein um eine Rotationsachse drehbar gelagerter Rotor mit wenigstens einem Förderelement zur Förderung von einer Flüssigkeit, insbesondere Blut, angeordnet ist,
eine Wandung, die die Pumpenkammer umgibt,
einen Stator mit mehreren Antriebsspulen zur Erzeugung eines Antriebsmagnetfeldes, das dazu eingerichtet ist, eine Rotationsbewegung des Rotors anzutreiben sowie
eine Steuereinrichtung, die dazu eingerichtet ist, Antriebsströme in den Antriebsspulen für den Rotationsantrieb des Rotors zu erzeugen, wobei der Rotor ein wenigstens teilweise elektrisch leitendes Target in Form eines Körpers aufweist, der von Mess-Wechselmagnetfeldern durchsetzbar ist, die durch eine Wechselmagnetfeldquelle, insbesondere eine oder mehrere Erregungsspulen erzeugt werden, dass die Pumpe eine oder mehrere Empfangsspulen aufweist und dass die Steuereinrichtung dazu eingerichtet ist, die durch die Mess- Wechselmagnetfelder in einer oder mehreren Empfangsspulen erzeugten und von der Wechselwirkung des Targets mit den Empfangsspulen abhängigen Wechselströme und Wechselspannungen zu messen, aus den jeweiligen Wechselspannungen und Wechselströmen komplexe Kenngrößen, insbesondere komplexe Widerstände einer oder mehrerer Empfangsspulen zu ermitteln und aus diesen eine Position des Rotors zu ermitteln, und wobei die Steuereinrichtung dazu eingerichtet ist, in einer Anzahl n von Erregungsspulen , wobei n größer als 1 ist, jeweils periodische Mess-Wechselspannungen zu erzeugen, die um eine Phasendifferenz von 360 Grad/n gegeneinander versetzt sind und die in den Empfangsspulen jeweils fließenden Mess-Wechselströme zu messen, und aus den jeweiligen Mess-Wechselspannungen und Mess-Wechselströmen komplexe Kenngrößen, insbesondere komplexe Widerstände, der Antriebsspulen zu ermitteln und aus einer Verknüpfung dieser Kenngrößen eine Position des Rotors zu ermitteln.

Eine Verknüpfung der Kenngrößen kann grundsätzlich eine Addition der Kenngrößen umfassen, wobei die Kenngrößen beispielsweise auch unterschiedlich gewichtet sein können.

Als Erregungsspulen zum Erzeugen eines Magnetfeldes, das mit dem Target des Rotors wechselwirkt, können auch einige oder alle Antriebsspulen dienen. Diese können zudem auch wenigstens teilweise als Empfangsspulen dienen. Dies hat den Vorteil, dass die Bedingungen der Erzeugung des oder der Magnetfelder einschließlich der Geometrie genau bekannt sind und dass die Mess-Wechselspannungen, die an den einzelnen Antriebsspulen anliegen, passend gestaltet werden können. Werden beispielsweise zwei oder drei Antriebsspulen genutzt und wird in jede der Antriebsspulen ein periodisches Wechselspannungssignal eingespeist, wobei die Wechselspanungssignale im Fall von zwei Spulen um 360 Grad/2 = 180 Grad, im Fall von drei Antriebsspulen um 360 Grad/3 = 120 Grad gegeneinander phasenverschoben sind, so können die mit den Mess- Wechselspannungen und den Mess- Wechselströmen ermittelten komplexen Widerstandswerte im komplexen Zahlenraum zur weiteren Auswertung, falls sinnvollmit einer passenden Gewichtung, addiert werden.

Die Antriebsspulen müssen nicht als einzelne Spule ausgeführt sein, sondern können auf mehrere in Reihe oder parallel geschaltete Spulen aufgeteilt sein. Dies tritt insbesondere bei Poolpaarzahlen > 1 auf.

Geht man von der Annahme aus, dass eine externe elektrische oder magnetische Störung alle für die Messung verwendeten Antriebsspulen gleichzeitig und in gleichem Maß trifft und beeinflusst, so ist die jeweilige Beeinflussung der ermittelten komplexen Widerstände durch einen Störimpuls bei jeder der Spulen im komplexen Zahlenraum um jeweils 360 Grad/n gedreht, so dass die Beiträge der Störimpulse in den verschiedenen verwendeten Spulen sich bei Addition der komplexen Widerstandswerte zu Null kompensieren. Externe Störimpulse können somit durch die beschriebene Summenbildung eliminiert werden.

Die Störkompensation kann auch schon bei der Spannungs- oder Strommessung angewendet werden. Hierzu werden die komplexen Messgrößen um die Phasenverschiebung der Erregung zurückgedreht, für n=3 beispielsweise um 0 Grad, 120 Grad und 240 Grad, und anschließend geometrisch im komplexen Zahlenraum addiert.

Eine weitere mögliche Realisierung der Pumpe kann vorsehen, dass die Steuereinrichtung dazu eingerichtet ist, Fehler bei der Erfassung von Mess-Wechselspannungen und -strömen zu detektieren und bei einer nicht ordnungsgemäßen Funktion der Messung oder der Bestimmung einer komplexen Kenngröße, insbesondere eines komplexen Widerstandes in einer der Erregungsspulen und/oder der Empfangsspulen die Anzahl oder die Auswahl oder die Gewichtung der Erregungsspulen und/oder der Empfangsspulen, die zur Messung der einer komplexen Kenngröße verwendet und mit einer Mess- Wechselspannung beaufschlagt werden, zu ändern und insbesondere in einer Anzahl m von Erregungsspulen jeweils periodische Mess-Wechselspannungen zu erzeugen, die um eine Phasendifferenz von 360 Grad/m gegeneinander versetzt sind, wobei m gleich n oder ungleich n ist.

Durch diese Lösung lassen sich Fehler und Schäden der Messvorrichtung einfach ohne einen äußeren Eingriff oder eine Reparatur beheben, ohne die Vorteile bei der Verwendung mehrerer Erregungs- und/oder Empfangsspulen für die Messzwecke zu verlieren und es können sehr wirksam Redundanzen vorgesehen und genutzt werden. Sind mehr als 3 Antriebsspulen vorhanden, so können diese beispielsweise alle als Erregungsspulen genutzt werden und die Mess-Wechselspannungen können passend gegeneinander versetzt werden. Fällt dann eine Spule oder eine Zuleitung aus, kann die Zahl der für die Messung verwendeten Spulen um 1 verringert und der Phasenversatz der verbleibenden Mess-Wechselspannungen neu festgelegt werden. Werden von Anfang an nicht alle Antriebsspulen für die Messung verwendet, so können ausgefallene Spulen durch andere, bisher nicht verwendete Spulen ersetzt werden oder ihre Messbeiträge können geringer gewichtet werden als die Beiträge der voll funktionsfähigen Spulen. Generell können eine, mehrere oder alle Erregungsspulen mit den Empfangsspulen und/oder mit den Antriebsspulen identisch sein Eine, mehrere oder alle Empfangsspulen können auch mit Antriebsspulen identisch sein, unabhängig davon, ob die Erregungsspulen Antriebsspulen oder separate Spulen oder Erzeuger für Mess- Wechselspanungsfelder sind.

Wie oben bereits angedeutet, können statt des Weglassens oder Ersetzens von fehlerbehafteten Empfangsspulen bei der Auswertung diese auch bei einer Verknüpfung, insbesondere einer Summenbildung oder Durchschnittsberechnung der Werte der Spulen weniger stark oder gar nicht gewichtet werden. Insgesamt können somit die Messwerte der einzelnen Phasen oder Empfangsspulen bei der Verknüpfung unterschiedlich gewichtet sein.

Es kann zu diesem Zweck auch vorgesehen sein, dass die Steuereinrichtung dazu eingerichtet ist, die Erregungsspulen, beispielsweise Antriebsspulen, in denen Mess-Wechselspannungen erzeugt werden, und/oder die Empfangsspulen, auf die Funktion der Erzeugung und Messung der Mess-Wechselspannung sowie die Messung des Mess-Wechselstroms und die Verarbeitung dieser Messgrößen mit einer Mess-Kontrollvorrichtung zu überwachen. Hierzu können regelmäßig in den Erregungsspulen Kontrollsignale erzeugt und es kann ihre magnetische Wirkung in Empfangsspulen überprüft werden oder die im normalen Messbetrieb erfassten Signale können auf Plausibilität geprüft werden. Die Signale der Empfangsspulen können auch beispielsweise auf das Auftreten von unerwarteten Signalformen, insbesondere unerwarteten Frequenzen, überprüft werden, die Fehler anzeigen können.

Die Fehlerdetektion kann beispielsweise dadurch erfolgen, dass die Steuereinrichtung eine Fehlerdetektionseinrichtung aufweist, die Messergebnisse der einzelnen Antriebsspulen laufend miteinander vergleicht und bei nicht plausiblen Abweichungen der Messergebnisse einer Spule von den Ergebnissen der anderen Spule, beispielsweise von einem ermittelten Durchschnittswert eines komplexen Widerstandswertes oder der Rotorposition die betreffende Antriebsspule von der weiteren Anwendung bei der Positionsmessung ausschließt oder bei der Berechnung ihre Gewichtung herabsetzt.

Die Pumpe kann zudem derart gestaltet sein, dass das Target als ein fest mit dem Rotor verbundener Körper, insbesondere als Scheibe oder als Ring, ausgebildet ist, der mit dem Rotor um dessen Rotationsachse rotiert und in azimutaler Richtung bezüglich der Rotorachse betrachtet einen oder mehrere erste Bereiche mit einer ersten elektrischen Leitfähigkeit und einen oder mehrere zweite Bereiche mit einer von der ersten elektrischen Leitfähigkeit verschiedenen zweiten elektrischen Leitfähigkeit aufweist oder dass der Rotor wenigstens teilweise aus einem elektrisch leitenden Werkstoff besteht und das Target durch einen elektrisch leitfähigen Teil des Rotors gebildet ist.

Auch in dem Fall, dass der Rotor wenigstens teilweise aus einem elektrisch leitenden Werkstoff besteht und das Target durch einen elektrisch leitfähigen Teil des Rotors gebildet ist, kann vorgesehen sein, dass das Target in azimutaler Richtung bezüglich der Rotorachse betrachtet einen oder mehrere erste Bereiche mit einer ersten elektrischen Leitfähigkeit und einen oder mehrere zweite Bereiche mit einer von der ersten elektrischen Leitfähigkeit verschiedenen zweiten elektrischen Leitfähigkeit aufweist. Diese verschiedenen Leitfähigkeitsbereiche des Targets in Azimutalrichtung können bei allen Gestaltungen des Targets beispielsweise in bestimmten Radien oder Radius-Intervallen, also ringförmigen, die Rotorachse umgebenden Bereichen gegeben sein.

Der Rotor kann beispielsweise in dem Pumpenraum rotieren und derart gelagert sein, dass seine Stirnseite sich vor einer Wand der Pumpenkammer befindet. Der Rotor kann dann an seiner Stirnseite das Target tragen.

Sind außen vor der Wand der Pumpenkammer Antriebsspulen des Pumpenmotors im Rahmen des Stators angeordnet, so beeinflusst das Target die Impedanz der Antriebsspulen, die die Erregungs- und/oder Empfangsspulen bilden können oder alternativer Erregungs/Empfangsspulen. Das Target bildet somit bei einer Erzeugung einer passenden magnetischen Erregung zusammen mit einer oder mehreren Spulen einen Wirbelstromsensor. Werden magnetische Wechselfelder, beispielsweise durch eine oder mehreren Erregungsspulen, im Bereich der Pumpe oder in der Pumpe selbst erzeugt, so werden in den Empfangsspulen Ströme und/oder Spannungen durch Induktion erzeugt, die als Mess-Ströme und Mess-Spannungen bei der Erregerfrequenz ausgewertet werden können, wobei aus diesen Größen jeweils ein komplexer Widerstand einer Spule ermittelt werden kann. Dieser komplexe Widerstand ist von der Wechselwirkung der jeweiligen Spule mit dem Target abhängig und die Bestimmung des Widerstandes ermöglicht somit beispielsweise die Bestimmung eines Abstandes des Targets von der jeweiligen Spule und damit auch die Bestimmung der Position des Rotors. Als Erregungsspulen und auch als Empfangsspulen können grundsätzlich in allen im vorliegenden Text beschriebenen Ausführungsformen alle Spulen des Rotors und des Stators der Pumpe, einschließlich der Magnetlagerspulen und der Antriebsspulen, genutzt werden. Es können somit die Empfangsspulen ganz oder teilweise mit den Erregungsspulen identisch sein und/oder eine oder mehrere Antriebsspulen können als Empfangsspulen und/oder als Erregungsspulen genutzt werden. Sind die Erregungs- und Empfangsspulen unabhängig von den Antriebsspulen und werden nur als Sensoren verwendet, dann werden in vielen Fällen auch nur die induzierte Spannung oder der dadurch fließende Strom gemessen und aus diesen wird, beispielsweise unter Berücksichtigung der Phasenverschiebung von Strom und Spannung, ein Abstand zu dem Target bestimmt. Eine konkrete Widerstandsbestimmung ist bei einem externen Sender, das heißt bei der Nutzung von außerhalb der Antriebsspulen oder außerhalb des Motors erzeugten Signalen für eine Induktivitätsmessung, oft nicht vorhanden. Im Zusammenhang dieses Textes wird an vielen Stellen von der Bestimmung eines komplexen Widerstandes von Spulen gesprochen, wobei jedoch auch jeweils jede andere aus dem Mess-Wechselstrom und der Mess- Wechselspannung bestimmbare, insbesondere komplexe, Größe mit umfasst sein soll, die von dem Abstand der jeweiligen Spule zum Target abhängig ist.

Die besondere Gestaltung des Targets ermöglicht alternativ oder gleichzeitig die Bestimmung einer Axial- oder Radialposition eines Rotors oder eines auf diesem befestigten Targets einerseits und andererseits die Bestimmung einer Winkelposition und auch einer Rotationsgeschwindigkeit des Rotors durch eine Analyse der periodischen Änderung der Messgrößen mit der Rotationsfrequenz des Rotors oder einem Vielfachen dieser Frequenz. Dabei kann die Form und Beschaffenheit des Targets einfach gestaltet sein und dieses kann in einfacher weise an dem Rotor befestigt werden. Es ist dabei weder ein Kippen des Targets gegen die Rotationsachse noch eine Justage in lateraler Richtung in Bezug auf den Rotor notwendig und es muss bei Verwendung eines einstückigen Targets auch nur ein einziger Körper mit dem Rotor verbunden werden.

Es kann konkret bei der Gestaltung des Targets vorgesehen sein, dass der oder die zweiten Bereiche des Targets eine andere Materialstärke oder Materialzusammensetzung aufweisen als die ersten Bereiche oder dass die zweiten Bereiche des Targets eine oder mehrere Ausnehmungen aufweisen.

Die Formulierung, dass das Target in azimutaler Richtung bezüglich der Rotorachse betrachtet einen oder mehrere erste Bereiche mit einer ersten elektrischen Leitfähigkeit und einen oder mehrere zweite Bereiche mit einer von der ersten elektrischen Leitfähigkeit verschiedenen zweiten elektrischen Leitfähigkeit aufweist, kann einerseits bedeuten, dass das Target in einzelnen Bereichen in Azimutalrichtung begrenzte Durchbrechungen, Ausnehmungen oder Ausdünnungen aufweist, die sich über einen bestimmten begrenzten Umfangsbereich oder azimutalen Winkelbereich erstrecken. Somit werden in diesem Zusammenhang auch die Durchbrechungen und Ausnehmungen des Targets als Bereiche des Targets bezeichnet, soweit sie klar zu der Gesamtgestalt des Targets gehören und beispielsweise innerhalb einer erkennbaren Gesamtkontur oder einhüllenden Außenform des Targets liegen. Andererseits können auch in solchen Bereichen andere Materialien oder Materialzusammensetzungen des Targets vorgesehen sein als in anderen Bereichen. Die Bereiche mit verschiedener Leitfähigkeit können einander in Azimutalrichtung des Targets auch mehrfach abwechseln. In diesem Fall werden die magnetischen Wechselwirkungen des Targets mit einer stationären Empfangsspule mit einem Vielfachen der Rotationsgeschwindigkeit moduliert. Besteht das Target aus verschiedenen Materialien mit unterschiedlicher elektrischer Leitfähigkeit und ohne Ausnehmungen, so können Unwuchten vermieden oder verringert werden, insbesondere, wenn die Materialien eine ähnliche Dichte aufweisen.

Durch die beschriebene Konstellation lässt sich durch die Positionsbestimmung insbesondere die Position eines Rotors eines Axialflussmotors in Axialrichtung bezüglich seiner Rotationsachse ermitteln und damit auch in vielen Fällen regeln. In einem Radialflussmotor kann mithilfe eines ringförmigen Targets die Rotorposition in einer oder mehreren radialen Richtungen bestimmt werden.

Eine Axialbewegung des Rotors kann beispielsweise bei in Axialrichtung fördernden Rotoren von der Pumpenlast abhängen und ein Axiallager belasten. Bei einem magnetisch gelagerten Rotor kann die Axialposition auch instabil sein, so dass sich eine bestimmte Axialposition nur durch Regelung halten lässt. Handelt es sich beispielsweise bei dem Axiallager um ein aktives magnetisches Lager, so kann dieses auch in Abhängigkeit von der ermittelten Rotorposition geregelt werden.

Die beschriebene Ausbildung einer Wirbelstromsensor- Konstellation, die auch durch die Wandung eines Pumpenraums hindurch funktioniert, bietet besondere Vorteile, ebenso wie die Verwendung einer oder mehrerer Antriebsspulen als Erregungs- und/oder Empfangsspulen, deren Verluste, bzw. deren Dämpfung der Induktivität als ein Maß für eine Relativposition eines Rotors oder eines auf ihm angeordneten Targets zur jeweiligen Spule dient. Auf diese Weise sind keine gesonderten Empfangsspulen zur Messung notwendig und Anschlüsse sowie gesonderte Zuleitungen für diese entfallen ebenfalls. Die Mess- und Auswertungsprozesse können in einfacher Weise in die Steuereinrichtung, beispielsweise eine Steuerelektronik und/oder eine Steuerungssoftware der Pumpe mit integriert werden. Eine kleine, raumsparende Bauweise, die sich auf diese Weise erreichen lässt, ist insbesondere für implantierbare Blutpumpen vorteilhaft.

Die Steuereinrichtung dient zudem in einigen Fällen dazu, die Antriebsspulen anzusteuern und Antriebs- Magnetfelder in passender Weise zu erzeugen, um bestimmte Drehzahlen der Pumpe zu erreichen oder magnetische Lagerkräfte zu erzeugen.

In einer weiteren Implementierung der Pumpe kann vorgesehen sein, dass der durch die Antriebsspulen erzeugte magnetische Fluss des Motors im Target überwiegend in Axialrichtung des Motors verläuft (es handelt sich dann um einen Axialflussmotor) und das Target die Form einer ebenen Scheibe aufweist, deren Flächennormale in Richtung zu einer oder mehreren Empfangsspulen orientiert ist oder dass der durch die Antriebsspulen erzeugte magnetische Fluss des Motors im Target überwiegend in Radialrichtung des Motors verläuft (es handelt sich dann um einen Radialflussmotor) und das Target die Form eines in Umfangsrichtung des Rotors umlaufenden Rings aufweist und eine oder mehrere Empfangsspulen radial außerhalb des Rotors an dessen äußerem Umfang angeordnet sind.

Bei einem Axialflussmotor kann vorgesehen sein, dass das Target die Form einer ebenen Scheibe aufweist, wobei die Flächennormale wenigstens einer der Flachseiten der Scheibe parallel zur Rotationsachse orientiert ist. In vielen Fällen wird dabei eine planparallele Scheibe oder Platte als Target verwendet werden. Ein solches scheibenförmiges Target kann jedoch auch gegenüber der Rotationsachse gekippt sein. In einem solchen Fall ist dann der magnetische Fluss mit der Rotationsfrequenz des Rotors moduliert, wobei dennoch sowohl die Axialposition als auch die Winkelposition des Rotors ermittelbar sind.

Ist das Target nicht gegenüber der Rotationsachse geneigt, so rotiert es mit dem Pumpenrotor in einem gleichmäßigen Abstand von den üblicherweise symmetrisch um die Rotationsachse angeordneten Antriebsspulen, so dass bei einem ideal rotationssymmetrischen Target alle Empfangsspulen bei der Rotation des Targets ständig demselben Einfluss des Targets unterliegen. Dieser hängt dann im Wesentlichen von einer veränderlichen Axialposition des Targets und somit des Rotors ab.

Das Taget kann aber auch als Ring oder Band um den Rotor herum ausgebildet sein, insbesondere dann, wenn die Antriebsspulen des Motors radial um den Rotor herum angeordnet sind. Mit einer derartigen Anordnung können Rotorunwucht und Axialschlag des Rotors im laufenden Betrieb detektiert werden. Ein derartiges kreisringförmiges oder torusförmiges Target wird in vielen Fällen konzentrisch zum Rotor angeordnet sein.

Grundsätzlich und für alle beschriebenen Ausführungsformen kann vorgesehen sein, dass das Target nicht homogen bezüglich der Rotationsachse ausgebildet ist, insbesondere wenigstens eine nicht zentrische Ausnehmung aufweist, die sich in der Umfangsrichtung um die Rotationsachse herum über einen begrenzten Winkelbereich erstreckt. Das Target kann ebenfalls, um einen ähnlichen Effekt zu erzielen, wenigstens einen nicht zentrischen Bereich aufweisen, in dem die elektrische Leitfähigkeit geringer ist, als in den übrigen Bereichen. Dies kann beispielsweise dadurch realisiert sein, dass das Material oder die Materialzusammensetzung sich in den genannten nicht zentrischen Bereichen von dem Material oder der Materialzusammensetzung in den übrigen Bereichen des Targets unterscheidet. Beispielsweise kann das Target aus Kupfer bestehen und Ausnehmungen aufweisen oder das Target kann insgesamt kreisscheibenförmig sein und neben Bereichen aus Kupfer, aus einem anderen Metall oder aus einem elektrisch leitenden oder mit Metallpartikeln gefüllten Kunststoff weitere Bereiche aufweisen, die aus einem Kunststoff mit geringer Leitfähigkeit oder einem sonstigen Isolierwerkstoff bestehen.

In diesem Fall ändert sich gemäß den obigen Ausführungen der Einfluss, den das Target auf jede der für die Abstandsmessungen verwendeten Empfangsspulen hat, periodisch mit der Rotation des Targets/Rotors relativ zu den Empfangsspulen. Bei Verwendung eines derartigen nicht homogenen Targets lässt sich somit durch die Auswertung der erfassten Dämpfung der Induktivitäten der Antriebsspulen neben der Axialposition oder der Radialposition auch die Rotationswinkelposition des Rotors jederzeit ermitteln.

In einer weiteren Ausführungsform kann vorgesehen sein, dass die Empfangsspulen auf der Außenseite der Wandung der Pumpenkammer angeordnet sind und dass die Wandung der Pumpenkammer mehr als 25% des Mess-Wechselmagnetfeldes transmittiert und das Target mehr als 25% des Mess-Wechselmagnetfeldes reflektiert oder absorbiert. Beispielsweise kann das Material der Wandung Titan oder eine Titanlegierung sein und das Material des Targets Kupfer oder eine Kupferlegierung.

In einer alternativen Betrachtungsweise kann vorgesehen sein, dass die Empfangsspulen auf der Außenseite der Wandung der Pumpenkammer angeordnet sind und dass die Wandung der Pumpenkammer aus einem ersten Material und das Target überwiegend aus einem zweiten Material besteht, wobei das zweite Material bei der Frequenz der Mess-Wechselspannung einen geringeren elektrischen Widerstand aufweist als das erste Material und wobei insbesondere das erste Material Titan oder eine Titanlegierung ist und das zweite Material Kupfer oder eine Kupferlegierung. Das zweite Material weist dann bei der Frequenz der Mess-Wechselspannung eine größere Eindringtiefe des Messsignals auf als das erste Material.

Grundsätzlich können sowohl das erste Material als auch das zweite Material ein Metall oder ein nicht metallisches, elektrisch besser oder schlechter leitendes Material sein. Die Beeinflussung der Dämpfung oder Herabsetzung der Induktivität der Antriebsspulen durch das Target, das sich in dem Pumpenraum auf dem Rotor befindet, wirkt bei geeigneter Wahl der Materialien und der Frequenz der Mess- Wechselspannung auch durch das Material der Wandung des Pumpenraums hindurch. Die Voraussetzung dafür ist, dass bei der Frequenz der Mess- Wechselspannung durch die Antriebsspulen im Material des Targets deutlich stärkere Wirbelströme erzeugt werden als in dem Material der Wandung des Pumpenraums. Zu diesem Zweck kann, falls die Wandung des Pumpenraums aus einer Titanlegierung besteht, wie sie bei medizinischen Implantaten häufig verwendet wird, das Target vorteilhaft beispielsweise aus Kupfer oder einer Kupferlegierung bestehen. Die Pumpenwandung kann jedoch ebenso aus einem nicht elektrisch leitenden Kunststoff bestehen. Die in der Pumpenraumwandung erzeugten Wirbelströme wirken bei den Messungen der Induktivitätsänderungen der Spulen als Messfehler und werden auf diese Weise möglichst gering gehalten. Die Wirbelströme in der Wandung schirmen das Target von der Messspule ab und erzeugen somit sowohl ein Widerstandsoffset als auch eine Verringerung der Sensitivität. Diese Wirbelströme sind daher entweder klein oder in allen Phasen, das heißt in allen Empfangsspulen, möglichst gleich zu halten.

Außerdem kann vorgesehen sein, dass die Steuereinrichtung dazu eingerichtet ist, in einer oder mehreren der Antriebsspulen jeweils periodische Mess-Wechselspannungen mit einer Frequenz zwischen 20kHz und 2MHz, insbesondere zwischen 50kHz und 100 kHz, zu erzeugen.

Dieser Frequenzbereich ist besonders bei einer Pumpenraumwandung aus Titan oder einer Titanlegierung und einem Target aus Kupfer vorteilhaft, weil in diesem Frequenzbereich die im Kupfer erzeugten Wirbelströme ausreichend groß und die in dem Titanwerkstoff erzeugten Wirbelströme deutlich kleiner sind.

Die Erfindung bezieht sich außer auf eine Pumpe der oben beschriebenen Art zudem auf ein Verfahren zum Betrieb einer Pumpe, insbesondere einer Blutpumpe, die einen in einer Pumpenkammer drehbar gelagerten und an-treibbaren Rotor sowie ein mit dem Rotor verbundenes Target und mehrere in einem Stator angeordnete Antriebsspulen aufweist, wobei das Verfahren vorsieht, dass
durch zwei oder mehr Erregungsspulen Mess-Wechselmagnetfelder erzeugt werden, die das Target und wenigstens eine Empfangssspule durchsetzen und dass eine Steuereinrichtung die durch die Mess- Wechselmagnetfelder erzeugten und von der Wechselwirkung des Targets mit den Empfangsspulen abhängigen Mess-Wechselströme und Mess-Wechselspannungen in einer oder mehreren Empfangsspulen erfasst, aus den jeweiligen Mess-Wechselspannungen und Mess-Wechselströmen komplexe Kenngrößen, insbesondere komplexe Widerstände einer oder mehrerer Empfangsspulen ermittelt und diese zur Ermittlung der Position des Rotors miteinander verknüpft, wobei eine Anzahl von n Erregungsspulen verwendet werden, wobei n wenigstens zwei beträgt, wobei in diesen jeweils durch die Steuereinrichtung periodische Mess-Wechselspannungen erzeugt werden, die um eine Phasendifferenz von 360 Grad/n gegeneinander versetzt sind.

Die Vorteile dieses Verfahrens sind bereits im Rahmen der Beschreibung der erfindungsgemäßen Pumpe weiter oben erläutert worden. Auch bei dem Verfahren können die Empfangsspulen identisch mit Antriebsspulen und/oder mit den Empfangsspulen sein. Ebenso können die Empfangsspulen identisch mit den Antriebsspulen und nicht identisch mit den Erregungsspulen sein.

Zudem kann in einer möglichen Ausführungsform des Verfahrens eine Anzahl von n der Antriebsspulen als Mess-Erregungsspulen verwendet werden, wobei n wenigstens zwei beträgt. In diesen können jeweils durch die Steuereinrichtung periodische Mess-Wechselspannungen erzeugt werden, die um eine Phasendifferenz von 360 Grad/n gegeneinander versetzt sind. Die in den Antriebsspulen jeweils fließenden Mess-Wechselströme können gemessen, sowie aus den jeweiligen Mess-Wechselspannungen und Mess-Wechselströmen komplexe Widerstände einer oder mehrerer Antriebsspulen ermittelt werden, und aus den komplexen Widerständen kann eine Position des Rotors ermittelt werden.

Bei einem derartigen Verfahren kann weiter vorgesehen sein, dass die mittels der Empfangsspulen ermittelten komplexen Kenngrößen, insbesondere komplexen Widerstandswerte miteinander verknüpft und aus der Verknüpfung wenigstens eine Position des Rotors ermittelt wird, wobei die Verknüpfung insbesondere die Bestimmung eines gewichteten Durchschnittswertes oder die Addition der einzeln gewichteten komplexen Kenngrößen, insbesondere der komplexen Widerstandswerte, umfasst und wobei weiter insbesondere Messfehler und Messstörungen detektiert die ermittelten Messwerte störungsbehafteter Empfangsspulen untergewichtet oder eliminiert werden.

Durch die Summierung oder Durchschnittswertbildungen der komplexen Widerstandswerte können infolge des Phasenversatzes universelle, das heißt, auf alle Phasen der Messung wirkende Störungen herausgefiltert werden. Die Verknüpfung der in den einzelnen Antriebsspulen ermittelten Positionswerte des Rotors kann zu einer Reduzierung von Messfehlern dienen.

Es kann weiter vorgesehen sein, das Target als ein fest mit dem Rotor verbundener Körper, insbesondere als Scheibe oder als Ring, ausgebildet ist, der mit dem Rotor um dessen Rotationsachse rotiert und in azimutaler Richtung bezüglich der Rotorachse einen oder mehrere erste Bereiche mit einer ersten elektrischen Leitfähigkeit und einen oder mehrere zweite Bereiche mit einer von der ersten elektrischen Leitfähigkeit verschiedenen zweiten elektrischen Leitfähigkeit aufweist und aus den mit der Rotationsfrequenz, oder Vielfachen der Rotationsfrequenz, des Rotors auftretenden Schwankungen der ermittelten Widerstands- und/oder Positionswerte eine Drehwinkelposition des Rotors ermittelt wird.

Bei Verwendung eines nicht homogenen Targets, beispielsweise einer ebenen Metallscheibe mit einem oder mehreren Ausschnitten, die sich über einen begrenzten Drehwinkelbereich erstrecken, funktioniert die Positionsbestimmung des Targets/Rotors in Axialrichtung noch ausreichend jeweils in den übrigen Rotationswinkelbereichen, in denen die Antriebsspulen den homogenen und beispielsweise gut elektrisch leitfähigen Bereichen des Targets gegenüberstehen und gleichzeitig erlaubt die Inhomogenität des Targets durch die Modulation der Wechselwirkung mit den Empfangsspulen entsprechend der Rotationsfrequenz des Rotors die Bestimmung einer Drehwinkelposition des Rotors.

Aus den Impedanz- oder Abstandsmessungen der einzelnen Phasen der Empfangsspulen kann die lineare Rotorposition, beispielsweise in Axial- oder Radialrichtung des Rotors, beispielsweise aus dem Maximum oder Minimum der Phasenimpedanzen (Impedanzen einzelner Empfangsspulen) bestimmt werden. Hierfür können die Ausnehmungen oder Bereiche mit verminderter Leitfähigkeit im Target so geformt sein, dass über einen bestimmten Drehwinkelbereich die Impedanz wenigstens einer Phase/Empfangsspule näherungsweise konstant ist, dadurch, dass einer oder mehreren Empfangsspule jeweils über einen bestimmten Drehwinkelbereich ein homogener Bereich des Targets gegenübersteht.

Alternativ kann das Target so geformt sein, dass der Impedanzverlauf jeder Phase näherungsweise sinusförmig verläuft. In diesem Spezialfall können jederzeit alle drei Phasen sowohl für die lineare Rotorlagemessung als auch für die Messung der Drehwinkelposition herangezogen werden. Die Impedanzen oder komplexen Messgrößen werden hierzu phasenversetzt aufaddiert, sodass sich in geometrischer Summe ein mit der Rotorrotation rotierender Phasor/Phasenzeigers ergibt. Das Argument, das heißt der Winkel, des Phasors lässt einen direkten Rückschluss auf die Rotationslage des Rotors zu. Der Betrag des Phasors lässt einen direkten Rückschluss auf die lineare Rotorposition zu.

Es kann weiter auch im Rahmen des Verfahren vorgesehen sein, bei einer nicht ordnungsgemäßen Funktion der Bestimmung eines komplexen Widerstandes in einer der Erregungsspulen und/oder der Empfangsspulen die Anzahl oder die Auswahl oder die Gewichtung der Erregungsspulen und/oder der Empfangsspulen, die zur Messung eines komplexen Widerstandes verwendet und mit einer Mess- Wechselspannung beaufschlagt werden, zu ändern und insbesondere in einer Anzahl m von Erregungsspulen jeweils periodische Mess-Wechselspannungen zu erzeugen, die um eine Phasendifferenz von 360 Grad/m gegeneinander versetzt sind, wo-bei m gleich n oder ungleich n ist. Es können somit fehlerbehaftete Erregungs- oder Empfangsspulen bei der Messung weggelassen und/oder auch weitere Erregungs/Empfangsspulen, soweit vorhanden, als Ersatz rekrutiert werden. Es können auch, wie bereits oben beschrieben, fehlerbehaftete Spulen oder allgemein Spulen, bei denen die Auswertung problematisch ist, weggelassen oder weniger stark gewichtet werden als die nicht fehlerbehafteten Spulen. Zu diesem Zweck kann die Steuereinrichtung die Messwerte der einzelnen Spulen regelmäßig auf Fehler oder Plausibilität prüfen.

Im Folgenden wird die Erfindung anhand von Ausführungsbeispielen in Figuren einer Zeichnung gezeigt und nachfolgend beschrieben.

Dabei zeigt
- Figur 1: schematisch eine Blutpumpe in einem Längsschnitt mit einem Rotor, einem Pumpenraum und Antriebsspulen,
- Figur 2: schematisch eine Antriebsspule und ihre Wechselwirkung mit einem Target,
- Figur 3: die Darstellung einer universellen Störung im Darstellungsraum der komplexen Widerstände,
- Figur 4: das Frequenzverhalten verschiedener Materialen mit unterschiedlichen Eindringtiefen in Bezug auf Wirbelströme,
- Figur 5: ein Phasenanschlussdiagramm eines Pumpenmotors bei der Verwendung von 3 Antriebsspulen für die Messung,
- Figur 6: eine Ansicht der Antriebsspulen und eines nicht homogenen Targets in Axialrichtung bezüglich der Rotationsachse,
- Figur 7: den Verlauf der ermittelten Widerstandswerte von 3 Antriebsspulen in Abhängigkeit vom Rotationswinkel bei Verwendung eines rotierenden, nicht homogenen Targets,
- Figuren 8, 9: weitere Ansichten von Antriebsspulen und nicht homogenen Targets in Axialrichtung bezüglich der Rotationsachse, sowie
- Figur 10: den Verlauf der elektrischen Messgrößen von drei Empfangsspulen bei einem gemäß Figur 9 oder ähnlich gestalteten Target.

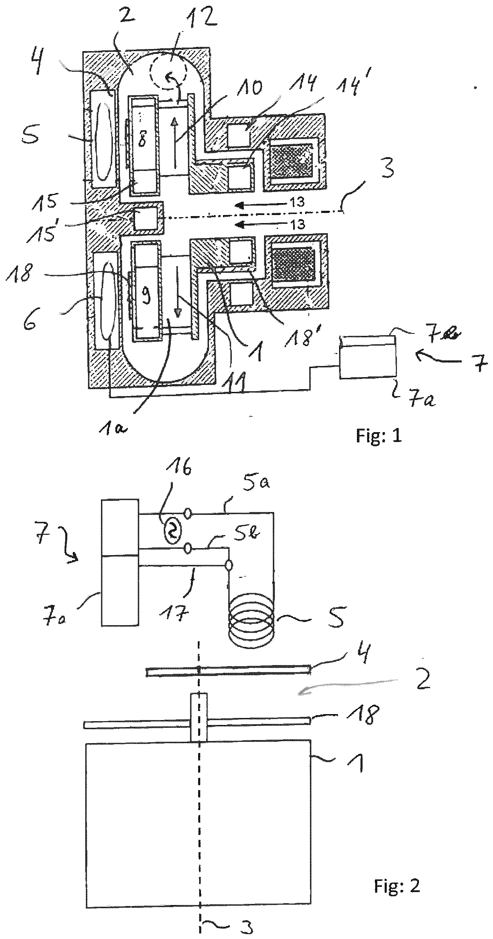

Figur 1 zeigt in einer Querschnittsansicht schematisch eine implantierbare Blutpumpe mit einem Rotor 1 der innerhalb einer Pumpenkammer 2 rotierend um eine Rotationsachse 3 gelagert ist. Durch eine Wandung 4 von der Pumpenkammer 2 getrennt sind außerhalb des Pumpenraums um die Rotationsachse 3 als Elemente eines Stators Antriebsspulen 5, 6 verteilt, die von einer Steuereinrichtung 7 angesteuert werden. Die Antriebsspulen 5, 6 erzeugen Magnetfelder, durch die mittels der Rotormagnete 8, 9 die Rotationsbewegung und in dem speziellen dargestellten Fall eine Axialbewegung des Rotors 1 angetrieben und/oder gesteuert wird.

Die zu fördernde Flüssigkeit, in dem konkret vorliegenden Fall Blut, wird entlang der Rotationsachse 3 in Richtung der Pfeile 13 zugeführt und durch die Rotationsbewegung des Rotors mit wenigstens teilweise in Radialrichtung verlaufenden Förderelementen 1a, 1b und die in diesen erzeugten Zentrifugalkräfte in Richtung der Pfeile 10, 11 in die Radialrichtung umgelenkt. Das Blut fließt dann über die Öffnung 12 in der Wandung 4 der Pumpenkammer/des Pumpengehäuses ab. Zudem sind in der Figur 1 magnetische Lager 14, 14' und 15, 15' dargestellt, die als Radiallager oder als kombinierte Radial/Axiallager ausgeführt sein können.

Die Steuereinrichtung 7 kann außer den Antriebsströmen zusätzlich die Mess-Wechselspannungen und Mess-Wechselströme erzeugen, die für die Ermittlung der komplexen Widerstandswerte der Antriebsspulen verwendet werden. Die Antriebsspulen 5, 6 bilden in diesem Fall sowohl die Erregungsspulen als auch die Empfangsspulen. Ein Target 18 kann als Scheibe auf den Rotormagneten 8,9 zwischen diesen und den Antriebsspulen 5,6 angeordnet werden. Weiter kann die Steuereinrichtung 7 die Mess- Wechselspannungen und Mess-Wechselströme in den Antriebsspulen erfassen und auswerten und aus den ermittelten komplexen Widerstandswerten oder einer alternativen Verknüpfung der gemessenen Strom- und Spannungsverläufe, beispielsweise unter Berücksichtigung der Phasenverschiebungen zwischen Strom- und Spannungsverläufen, eine Position des Targets und des Rotors in Axialrichtung des Rotors ermitteln. Die Steuereinrichtung 7 weist neben einer Auswerteeinheit 7a auch eine Mess-Kontrollvorrichtung 7b zur regelmäßigen Überprüfung und/oder Plausibilitätsprüfung der Messwerte auf, die dazu eingerichtet ist, fehlerhafte Spulen zu detektieren. Die Mess- Kontrollvorrichtung kann zu diesem Zweck einen Micro Controller zur Datenverarbeitung aufweisen oder ein neuronales Netz, das auf die Erkennung von Fehlern trainiert ist.

In einem anderen Fall, in dem die Antriebsspulen am Umfang des Rotors in Umfangsrichtung um diesen herum verteilt sind, kann ein Target auch die Form eines Torus/Kreisrings aufweisen und auf dem Rotor konzentrisch zu diesem befestigt sein. Als Beispiel ist ein ringförmiges, um den Rotor umlaufendes Target 18' in der Figur 1 dargestellt. In diesem Fall kann durch das beschriebene Messverfahren die Position des Rotors in Radialrichtung bestimmt werden.

In der Figur 2 ist schematisch ein Rotor 1 einer Pumpe dargestellt, dem eine einzelne Antriebsspule 5 stirnseitig gegenübersteht. Zwischen dem Rotor 1 und der Antriebsspule 5 liegt die Wand 4 der Pumpenkammer 2. Die Rotationsachse der Pumpe ist auch hier mit 3 bezeichnet.

Die Antriebsspule 5 wird mit einer Mess-Wechselspannung 16 an den Anschlüssen 5a, 5b beaufschlagt, die dem Antriebsstrom überlagert ist. Der Mess- Wechselstrom wird beispielsweise In über den Anschluss 17 gemessen.

In der Auswerteeinheit 7a der Steuereinrichtung 7 wird aus der Mess- Wechselspannung und dem Mess- Wechselstrom für die Frequenz der Mess-Wechselspannung ein komplexer Widerstandswert ermittelt. Dieser Widerstandswert hängt unter anderem von der Wechselwirkung der Antriebsspule 5 mit dem Target 18 ab, das als flache, auf dem Rotor befestigte und mit diesem rotierende Scheibe aus einem Kupfermaterial ausgebildet ist. Insbesondere hängt der Widerstandswert von dem Abstand zwischen der Antriebsspule und dem Target ab. In dem Target 18 werden durch das Mess-Magnetfeld/das magnetische Erregungsfeld Wirbelströme erzeugt, die den Strom- und Spanungsverlauf bei der Mess-Frequenz sowie, damit verknüpft, den komplexen Widerstandswert der Antriebsspule beeinflussen. Die Wand 4 der Pumpenkammer besteht in dem gezeigten Beispiel aus einer Titanlegierung, in der in einem passend gewählten Frequenzbereich nur vergleichsweise geringe Wirbelströme erzeugt werden, so dass die Wand 4 für die magnetischen Wechselfelder weitgehend durchlässig ist. Das Target kann beispielsweise aus Kupfer bestehen, so dass zusammen mit der Wand der Pumpenkammer aus einer Titanlegierung eine gute Materialpaarung entsteht.

In einer anderen Variante kann ein magnetisches Erregungsfeld auch durch ein anderes Element als die Antriebsspule(n) erzeugt werden, beispielsweise durch eine oder mehrere Spulen, die innerhalb der Pumpe oder an der Pumpe angeordnet sind und mit einer Mess- Wechselspannung versorgt werden können, wie beispielsweise eine Spule, die als Teil eines aktiven Magnetlagers ansteuerbar ist und als Erregungsspule fungiert, oder auch durch eine andere Quelle, die autonom ein magnetisches Wechselfeld erzeugt. In diesem Fall werden in einer oder mehreren Antriebsspulen, die dann beispielsweise als Empfangsspulen dienen, Mess- Wechselspannungen und Mess-Wechselströme in einem passenden Frequenzbereich gemessen und es wird für die vorliegende Frequenz ein komplexer Widerstandswert oder eine andere Verknüpfungsgröße aus dem Mess- Wechselstrom und der Mess-Wechselspannung und eine damit korrespondierende Rotorposition bestimmt.

Für die Zuordnung von Rotorpositionen zu den ermittelten komplexen Widerstandswerten können Eichtabellen oder eine kalibrierte digitale Umrechnungsfunktion dienen. Als Aspekte der ermittelbaren Rotorposition kommt zunächst eine Axialposition oder eine Abweichung von einer axialen Referenzposition in Frage, jedoch auch allgemein eine Position des Rotors in Axial- und Radialrichtung.

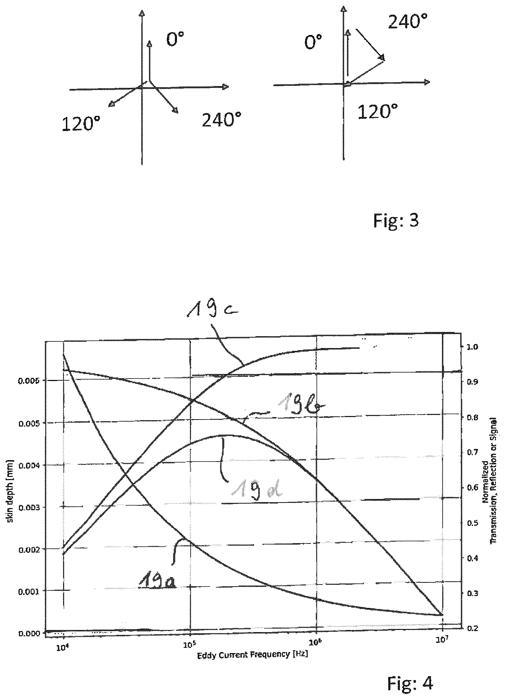

Es ist mit der beschriebenen Vorrichtung möglich, die Mess- Wechselspannung und den Mess- Wechselstrom in mehreren Antriebsspulen zu bestimmen und , wenn die Mess- Wechselspannungen aktiv in den Antriebsspulen erzeugt wird, die einzelnen Mess- Wechselspannungen, die typischerweise als Sinusspannung erzeugt werden, bei Verwendung von n Antriebsspulen bezüglich der Phasenlage um 360 Grad /n gegeneinander zu versetzen. Die Figur 3 zeigt beispielhaft die Situation bei Verwendung von n=3 Antriebsspulen und einer globalen, alle drei Spulen beeinflussenden Störung im komplexen Raum. Auf der linken Seite der Figur 3 ist der Zeiger dreifach für die Störung in den einzelnen Antriebsspulen gezeigt, die Phasenlage der Störungen ist in den einzelnen Spulen um jeweils 120 Grad gegeneinander verdreht.

Werden die drei ermittelten komplexen Widerstandswerte oder Messgrößen der drei beteiligten Antriebsspulen addiert, so ergibt sich gemäß dem Diagramm auf der rechten Seite der Figur 3, dass die Störung durch die Addition der drei Phasen zu Null kompensiert wird. Aus den störungskompensierten Summenwert kann die Position des Rotors zuverlässig bestimmt werden. Eine zusätzliche Kompensation kann sich bei vielen Pumpenkonstruktionen dadurch ergeben, dass eine Antriebsspule auf zwei oder mehr Wicklungen aufgeteilt ist die am Umfang des Pumpenstators symmetrisch verteilt sind, so dass viele externe Störungen auch bereits durch diese Symmetrie wenigstens teilweise kompensiert werden.

In der Figur 4 sind die Wirbelstromverhältnisse in Abhängigkeit von der Frequenz der magnetischen Wechselfelder dargestellt. Dabei bezeichnet die Kurve 19a die Eindringtiefe des Stroms in dem Titanmaterial TiAIV, die sich aufgrund der Stromverdrängung durch den Skin- Effekt einstellt. Die Kurve 19b stellt den frequenzabhängigen Verlauf der Transmission eines Wechselfeldes durch das Titanmaterial dar. Die Kurve 19c zeigt den an einem Kupfertarget reflektierten Anteil des magnetischen Wechselfeldes und die Kurve 19d stellt die gemessene Signalstärke infolge der Wechselwirkung des Wechselfeldes durch eine Titanwandung hindurch mit einem Kupfertarget dar, welche das Produkt aus 19b und 19c ist.

Damit ergibt sich eine gute Signalstärke im Frequenzbereich zwischen 20 kHz und 2Mhz, insbesondere zwischen 50kHz und 100kHz oder auch zwischen 50kHz und 200kHz.

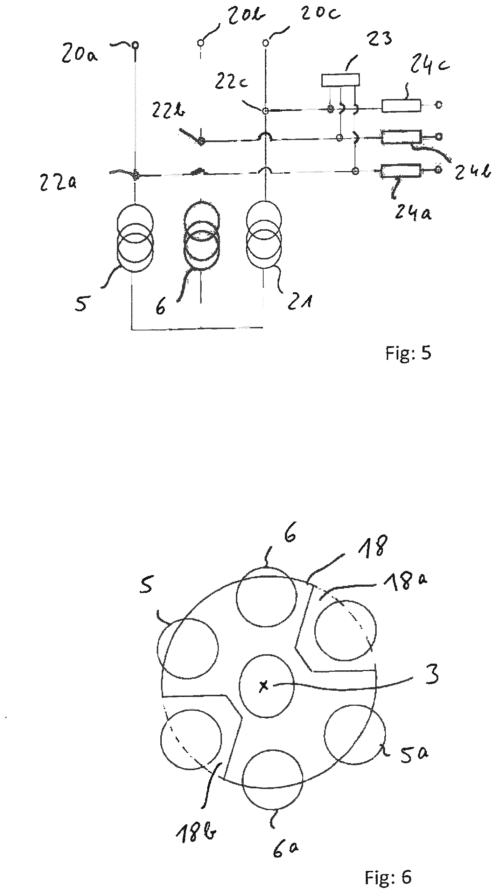

Die Figur 5 zeigt ein schematisches Phasenanschlussdiagramm eines dreiphasigen Elektromotors, der als Antrieb für eine Pumpe dient. Über die Anschlüsse 20a, 20b, 20c wird außer den Antriebsströmen in die Antriebsspulen 5, 6, 21 das Mess- Wechselspannungssignal eingespeist. Der Mess- Wechselstrom wird an den Anschlüssen 22a, 22b, 22c erfasst. In einem Frequenzmischer 23 wird den gemessenen Mess- Wechselspannungssignalen ein weiteres, relativ hochfrequentes Wechselspannungssignal, beispielsweise im Bereich von 50kHz, überlagert und in einem Tiefpass 24a, 24b, 24c wird aus dem Mischsignal jeweils ein niederfrequentes Signal ausgekoppelt, das dem Verlauf der gemessenen, nur wenig zeitlich veränderlichen Mess-Wechselströme entspricht.

Aus den Signalen von Mess- Wechselspannungen und Mess- Wechselströmen lassen sich dann die komplexen Widerstandswerte bei der Frequenz der Signale und nachfolgend die Rotorposition ermitteln.

Mischer 23 und Tiefpass 24a, 24b, 24c lassen für jede Phase in einem einzelnen analogen oder digitalen Filter vereinen. Digitale Filter, beispielsweise aufgebaut als FIR oder IIR Filter, können dabei besonders gute Filtereigenschaften aufweisen. Es können in die Tiefpassfunktion auch weitere Filterfunktionen integriert werden, um beispielsweise bekannte Störer wie eine PWM-(Pulsweitenmodulations)-Frequenz, Motorantriebsströme oder Lagerantriebsströme zu unterdrücken.

Die Figur 6 zeigt schematisch in einer stirnseitigen Ansicht, das heißt in Richtung parallel zur Rotorachse 3 eines Pumpenmotors gesehen, 3 Antriebsspulen 5, 6, 21, wobei bei jeder Antriebsspule 5, 6, 21 einander jeweils zwei Spulenhälften 5, 5a, 6, 6a am Umfang des Stators gegenüberstehen. Weiter ist in der Figur 6 ein kreisringscheibenförmiges Kupfertarget 18 dargestellt, das teilweise an seinem Umfang mit Ausschnitten/Ausnehmungen 18a, 18b versehen ist. Diese gelten in der Nomenklatur des vorliegenden Textes als Bereiche des Targets mit verringerter elektrischer Leitfähigkeit. Als Target wird dabei das gesamte kreisscheibenförmige Target verstanden, das durch die Ausnehmungen 18a, 18b vervollständigt ist. Die Kreisscheibenform bildet somit die Gesamtkontur oder Einhüllende des Targets. Die vervollständigte Kreisform am Umfang des Kupfertargets ist teilweise gestrichelt dargestellt, um die Ausschnitte besser sichtbar zu machen. Die beiden Ausschnitte 18a, 18b bilden Kreisringsegmente, die einander am Umfang des Kupfertargets gegenüberliegen. Der Einfluss des Kupfertargets auf den in den Antriebsspulen gemessenen komplexen Widerstand bei der Frequenz der Mess- Wechselspannung ist durch die Ausschnitte 18a, 18b stark von der Drehwinkelposition des Targets und damit des Rotors abhängig.

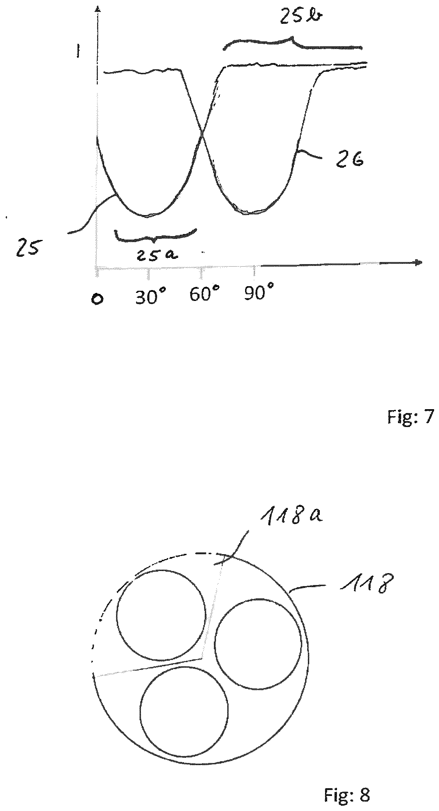

In der Figur 7 ist dargestellt, wie der gemessene Widerstandswert 25, 26 von zwei Antriebsspulen vom Drehwinkel des Targets/Rotors abhängt. Der Drehwinkel des Targets ist in dem Diagramm der Figur 7 auf der horizontalen Achse dargestellt, während auf der vertikalen Achse der Widerstand der Antriebsspulen dargestellt ist, der sich infolge der in dem Target jeweils fließenden Wirbelströme einstellt. Es ergeben sich Einbrüche /Reduzierungen 25a des Widerstandswertes, die sich für jede einzelne Antriebsspule jeweils im Abstand von 180 Grad des Rotordrehwinkels einstellen. Für zwei verschiedene von drei Phasen haben die Absenkungen jeweils einen Abstand von 60 Grad des Rotordrehwinkels. In den nahezu konstant verlaufenden Bereichen 25b - dargestellt am Beispiel des Widerstandsverlaufs 25- außerhalb der Absenkungen repräsentiert der dort gemessene Widerstandswert die tatsächliche Axialposition des Rotors. Damit lässt sich aus dem erfassten Verlauf der Widerstandswerte mit einem ausgeschnittenen Target auch die Drehwinkelposition des Rotors zusätzlich oder alternativ zu einer axialen Position des Rotors gut ermitteln.

Die Positionsermittlung des Rotors, die beispielsweise für die Steuerung eines aktiven magnetischen Axiallagers verwendet werden kann, kann somit mit der erforderlichen Genauigkeit und Zeitauflösung mit einem Minimum an zusätzlichem Hardwareaufwand ermöglicht werden.

In der Figur 8 ist eine weitere mögliche Gestaltung eines Targets 118 mit nur einer einzigen Ausnehmung 118a gezeigt. Dieses Target kann analog zu dem Target 18 an der Stirnseite eines Rotors befestigt werden.

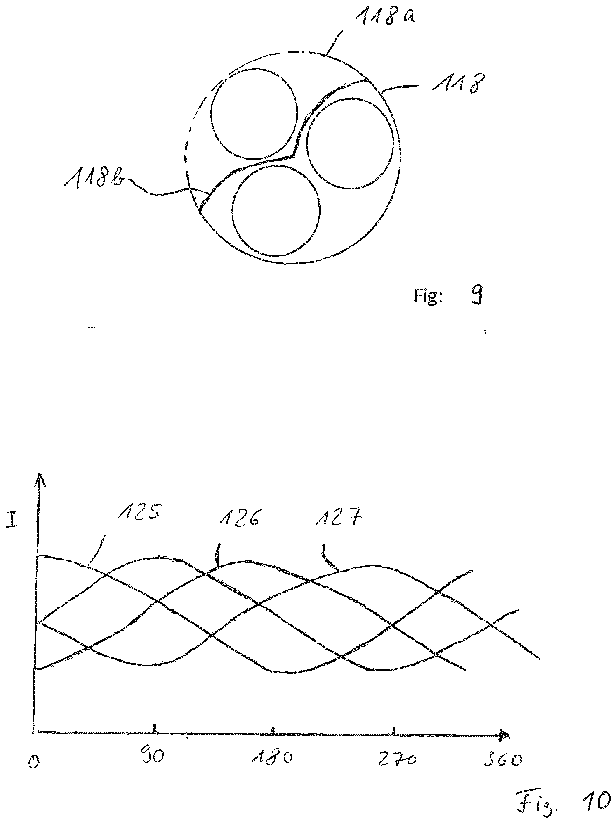

Das in der Figur 9 dargestellte Target unterscheidet sich von dem in Figur 8 gezeigten Target durch die geometrische Gestaltung des Randes der Ausnehmung 118a. Der Rand 118b der Ausnehmung 118a gemäß Figur 9 ist derart gestaltet dass sich, wenn drei stirnseitige Antriebsspulen des Motors als Erregungs- und Empfangsspulen benutzt werden, bei in Axialrichtung gleichbleibender Rotorposition für jede Spule ein sinusförmiger Verlauf der Widerstandswerte ergibt. Der Rand 118b kann zu diesem Zweck zweifach konvex gekrümmt gestaltet sein. Die sinusförmigen Verläufe 125, 126, 127 der einzelnen Spulen sind in der Figur 10 dargestellt.

## Patentansprüche

1. Pumpe, insbesondere Blutpumpe, umfassend
eine Pumpenkammer (2), in der ein um eine Rotationsachse (3) drehbar gelagerter Rotor (1) mit wenigstens einem Förderelement (1a, 1b) zur Förderung von einer Flüssigkeit, insbesondere Blut, angeordnet ist,
eine Wandung (4), die die Pumpenkammer umgibt,
einen Stator mit mehreren Antriebsspulen (5, 6, 21) zur Erzeugung eines Antriebsmagnetfeldes, das dazu eingerichtet ist, eine Rotationsbewegung des Rotors anzutreiben sowie
eine Steuereinrichtung (7), die dazu eingerichtet ist, Antriebsströme in den Antriebsspulen für den Rotationsantrieb des Rotors zu erzeugen,
wobei der Rotor ein wenigstens teilweise elektrisch leitendes Target (18, 18') in Form eines Körpers aufweist, der von Mess-Wechselmagnetfeldern durchsetzbar ist, die durch eine Wechselmagnetfeldquelle, insbesondere eine oder mehrere Erregungsspulen (5, 6, 21) erzeugt werden, dass die Pumpe eine oder mehrere Empfangsspulen aufweist und dass die Steuereinrichtung dazu eingerichtet ist, die durch die Mess- Wechselmagnetfelder in einer oder mehreren Empfangsspulen erzeugten und von der Wechselwirkung des Targets mit den Empfangsspulen abhängigen Wechselströme und Wechselspannungen zu messen, aus den jeweiligen Wechselspannungen und Wechselströmen komplexe Kenngrößen, insbesondere komplexe Widerstände einer oder mehrerer Empfangsspulen zu ermitteln und aus diesen eine Position des Rotors (1) zu ermitteln,
**dadurch gekennzeichnet, dass** die Steuereinrichtung (7) dazu eingerichtet ist, in einer Anzahl n von Erregungsspulen (5, 6, 21), wobei n größer als 1 ist, jeweils periodische Mess-Wechselspannungen zu erzeugen, die um eine Phasendifferenz von 360 Grad/n gegeneinander versetzt sind und die in den Empfangsspulen jeweils fließenden Mess-Wechselströme zu messen, und aus den jeweiligen Mess-Wechselspannungen und Mess-Wechselströmen komplexe Kenngrößen, insbesondere komplexe Widerstände, der Antriebsspulen zu ermitteln und aus einer Verknüpfung dieser Kenngrößen eine Position des Rotors (1) zu ermitteln.

2. Pumpe nach Anspruch 1, **dadurch gekennzeichnet, dass** die Steuereinrichtung (7) dazu eingerichtet ist, Fehler bei der Erfassung von Mess-Wechselspannungen und -strömen zu detektieren und bei einer nicht ordnungsgemäßen Funktion der Messung oder der Bestimmung einer komplexen Kenngröße, insbesondere eines komplexen Widerstandes in einer der Erregungsspulen (5, 6, 21) und/oder der Empfangsspulen die Anzahl oder die Auswahl oder die Gewichtung der Erregungsspulen und/oder der Empfangsspulen, die zur Messung der komplexen Kenngröße verwendet und mit einer Mess- Wechselspannung beaufschlagt werden, zu ändern und insbesondere in einer Anzahl m von Erregungsspulen jeweils periodische Mess-Wechselspannungen zu erzeugen, die um eine Phasendifferenz von 360 Grad/m gegeneinander versetzt sind, wobei m gleich n oder ungleich n ist.

3. Pumpe nach Anspruch 1 oder 2, **dadurch gekennzeichnet, dass** das Target als ein fest mit dem Rotor verbundener Körper, insbesondere als Scheibe oder als Ring, ausgebildet ist, der mit dem Rotor um dessen Rotationsachse rotiert und in azimutaler Richtung bezüglich der Rotorachse betrachtet einen oder mehrere erste Bereiche mit einer ersten elektrischen Leitfähigkeit und einen oder mehrere zweite Bereiche mit einer von der ersten elektrischen Leitfähigkeit verschiedenen zweiten elektrischen Leitfähigkeit aufweist oder dass der Rotor wenigstens teilweise aus einem elektrisch leitenden Werkstoff besteht und das Target durch einen elektrisch leitfähigen Teil des Rotors gebildet ist.

4. Pumpe nach Anspruch 3, **dadurch gekennzeichnet, dass** der oder die zweiten Bereiche des Targets (18, 18') eine andere Materialstärke oder Materialzusammensetzung aufweisen als die ersten Bereiche oder dass die zweiten Bereiche des Targets eine oder mehrere Ausnehmungen aufweisen.

5. Pumpe nach einem der Ansprüche 1 bis 4, **dadurch gekennzeichnet, dass** der durch die Antriebsspulen (5, 6, 21) erzeugte magnetische Fluss des Motors im Target überwiegend in Axialrichtung (3) des Motors verläuft und das Target (18) die Form einer ebenen Scheibe aufweist, deren Flächennormale in Richtung zu einer oder mehreren Empfangsspulen orientiert ist oder dass der durch die Antriebsspulen (5, 6, 21) erzeugte magnetische Fluss des Motors im Target überwiegend in Radialrichtung des Motors verläuft und das Target die Form eines in Umfangsrichtung des Rotors umlaufenden Rings aufweist und eine oder mehrere Empfangsspulen radial außerhalb des Rotors angeordnet sind.

6. Pumpe nach einem der Ansprüche 1 bis 5, **dadurch gekennzeichnet, dass** die Empfangsspulen (5, 6, 21) auf der Außenseite der Wandung (4) der Pumpenkammer (2) angeordnet sind und dass die Wandung der Pumpenkammer mehr als 25% des Mess-Wechselmagnetfeldes transmittiert und das Target mehr als 25% des Mess-Wechselmagnetfeldes reflektiert oder absorbiert und/oder dass die Wandung (4) der Pumpenkammer aus einem ersten Material und das Target (18, 18') überwiegend aus einem zweiten Material besteht, wobei das zweite Material bei der Frequenz der Mess-Wechselspannung einen geringeren elektrischen Widerstand aufweist als das erste Material und wobei insbesondere das erste Material Titan oder eine Titanlegierung ist und/oder das zweite Material Kupfer oder eine Kupferlegierung.

7. Pumpe nach einem der Ansprüche 1 bis 6, **dadurch gekennzeichnet, dass** die Steuereinrichtung (7) dazu eingerichtet ist, in einer oder mehreren der Erregungsspulen (5, 6, 21) jeweils periodische Mess-Wechselspannungen mit einer Frequenz zwischen 20kHz und 2MHz, insbesondere zwischen 50kHz und 100 kHz, zu erzeugen.

8. Verfahren zum Betrieb einer Pumpe, insbesondere einer Blutpumpe, die einen in einer Pumpenkammer (2) drehbar gelagerten und antreibbaren Rotor (1) sowie ein mit dem Rotor verbundenes Target (18, 18') und mehrere in einem Stator angeordnete Antriebsspulen (5, 6, 21) aufweist, **dadurch gekennzeichnet, dass**
durch zwei oder mehr Erregungsspulen Mess-Wechselmagnetfelder erzeugt werden, die das Target und wenigstens eine Empfangsspule durchsetzen und dass eine Steuereinrichtung (7) die durch die Mess-Wechselmagnetfelder erzeugten und von der Wechselwirkung des Targets mit den Empfangsspulen abhängigen Mess-Wechselströme und Mess-Wechselspannungen in einer oder mehreren Empfangsspulen erfasst, aus den jeweiligen Mess-Wechselspannungen und Mess-Wechselströmen komplexe Kenngrößen, insbesondere komplexe Widerstände, einer oder mehrerer Empfangsspulen ermittelt und diese zur Ermittlung der Position des Rotors miteinander verknüpft,
wobei eine Anzahl von n Erregungsspulen verwendet werden, wobei n wenigstens zwei beträgt, wobei in diesen jeweils durch die Steuereinrichtung periodische Mess-Wechselspannungen erzeugt werden, die um eine Phasendifferenz von 360 Grad/n gegeneinander versetzt sind.

9. Verfahren nach Anspruch 8, **dadurch gekennzeichnet, dass** die mittels der Empfangsspulen ermittelten komplexen Kenngrößen, insbesondere Widerstandswerte, miteinander verknüpft und aus der Verknüpfung wenigstens eine Position des Rotors ermittelt wird, wobei die Verknüpfung insbesondere die Bestimmung eines gewichteten Durchschnittswertes oder die Addition der einzeln gewichteten komplexen Messgrößen, insbesondere der komplexen Widerstandswerte, umfasst und wobei weiter insbesondere Messfehler und Messstörungen detektiert und die ermittelten Messwerte störungsbehafteter Empfangsspulen untergewichtet oder eliminiert werden.

10. Verfahren nach Anspruch 8 oder 9, wobei das Target (18, 18') als ein fest mit dem Rotor (1) verbundener Körper, insbesondere als Scheibe oder als Ring, ausgebildet ist, der mit dem Rotor um dessen Rotationsachse (3) rotiert und in azimutaler Richtung bezüglich der Rotorachse einen oder mehrere erste Bereiche mit einer ersten elektrischen Leitfähigkeit und einen oder mehrere zweite Bereiche mit einer von der ersten elektrischen Leitfähigkeit verschiedenen zweiten elektrischen Leitfähigkeit aufweist und aus den mit der Rotationsfrequenz, oder Vielfachen der Rotationsfrequenz, des Rotors (1) auftretenden Schwankungen der ermittelten Widerstands- und/oder Positionswerte eine Drehwinkelposition des Rotors ermittelt wird.
